(19) Europäisches Patentamt
European Patent Office
Office européen des brevets

(11) Numéro de publication: **0 191 669**
**A1**

(12) # DEMANDE DE BREVET EUROPEEN

(21) Numéro de dépôt: **86400092.2**

(22) Date de dépôt: **17.01.86**

(51) Int. Cl.[4]: **C 07 D 307/68**

(30) Priorité: **25.01.85 FR 8501039**

(43) Date de publication de la demande: **20.08.86**
**Bulletin 86/34**

(84) Etats contractants désignés: **AT BE DE FR GB IT NL SE**

(71) Demandeur: **RHONE-POULENC SPECIALITES CHIMIQUES, "Les Miroirs" 18, Avenue d'Alsace, F-92400 Courbevoie (FR)**

(72) Inventeur: **Reeb, Roland, 2, allée des Pléiades, Gressy en France F-77410-Claye Souilly (FR)**
Inventeur: **Chauvel, Bernard, 24, Chemin de la Commanderie, F-95120 Ermont (FR)**

(74) Mandataire: **Fabre, Madeleine-France et al, RHONE-POULENC INTERSERVICES Service Brevets Chimie 25, quai Paul Doumer, F-92408 Courbevoie Cédex (FR)**

(54) **Nouveaux esters insaturés de l'acide furoïque et procédé pour leur préparation.**

(57) Esters insaturés de l'acide furoïque de formule:

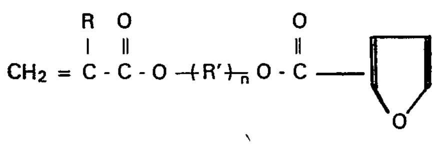

(I)

où

. R représente de l'hydrogène ou un groupe alkyle, R' un radical alkylène linéaire ou ramifié et n un nombre entier allant de 1 à 4.

Ils peuvent être préparés par action d'un acrylate ou alkylacrylate d'hydroxyalkyle de formule $CH_2=CR\text{-}COO-(R')_n OH$

Ils peuvent être utilisés comme monomères pour la préparation de polymères réticulables.

EP 0 191 669 A1

# NOUVEAUX ESTERS INSATURES DE L'ACIDE FUROIQUE ET PROCEDE POUR LEUR PREPARATION

La présente invention a pour objet de nouveaux esters insaturés de l'acide furoïque, à savoir les acrylates ou alkylacrylates de furoate d'alkyle et leur procédé de préparation.

On connaît notamment le vinyl 2-furoate et le méthyl-5 vinyl-2-furoate comme esters insaturés de l'acide furoïque ("Advances in Polymer Science" n° 25 - 1977 - GANDINI, "The Behaviour of Furan Derivatives in Polymerization Reactions" p.77) ; ces produits ne présentent que peu ou pas d'intérêt en tant que monomères.

Les esters insaturés de l'acide furoïque faisant l'objet de l'invention sont caractérisés en ce qu'ils ont pour formule :

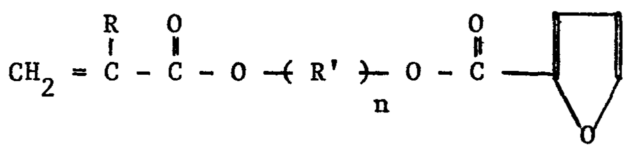 (I)

formule dans laquelle :

. R représente de l'hydrogène ou un groupe alkyle en $C_1$-$C_2$ et de préférence en $C_1$

. R' représente un radical alkylène linéaire ou ramifié en $C_1$-$C_5$ et de préférence en $C_2$-$C_4$

. n représente un nombre entier allant de 1 à 4 et de préférence égal à 1.

Ces esters insaturés de formule (I) peuvent être obtenus par réaction d'un acrylate, méthylacrylate ou éthylacrylate d'hydroxyalkyle de formule :

$$CH_2 = \overset{\displaystyle R}{\overset{|}{C}} - \overset{\displaystyle O}{\overset{\|}{C}} - O -\!\!\left( R' \right)_{n}\!\!- OH \qquad (II)$$

où R, R' et n ont la signification donnée ci-dessus, avec un halogénure de furoyle, les quantités de réactifs mises en oeuvre correspondant à un rapport molaire halogénure de furoyle/réactif de formule (II) compris entre la stoechiométrie et un excès de 10 % de l'un ou l'autre des réactifs par rapport à la stoechiométrie.

Parmi les réactifs de formule (II) pouvant être mis en oeuvre, on peut citer en particulier : les acrylates ou méthacrylates de 2-hydroxyéthyle, 2-hydroxypropyle, 2-hydroxy-1-méthyléthyle, 3-hydroxypropyle, 2-hydroxy-1-éthyléthyle, 4-hydroxybutyle ...

Parmi les halogénures de furoyle, on peut citer le bromure de furoyle et tout particulièrement le chlorure de furoyle.

Pour une bonne mise en oeuvre du procédé, ladite réaction est réalisée à une température comprise entre 0 et 40° C, de préférence entre 5 et 20° C, en présence d'un catalyseur piègeant l'hydracide formé et d'un solvant commun à l'halogénure de furoyle et au réactif de formule II.

Parmi les catalyseurs pouvant être mis en oeuvre, on peut citer les amines tertiaires ne possèdant pas d'hydrogène labile, telles que les trialkylamines présentant des radicaux alkyles en $C_1$-$C_6$, les N,N dialkylanilines présentant des radicaux alkyles en $C_1$-$C_2$, les alkylènepolyamines tertiaires (triéthylène diamine, N,N,N',N'-tétraméthyléthylènediamine, N, N, N', N'-tétraméthyl--1,3 butanediamine, ...), les amines tertiaires aromatiques telles que la pyridine, les méthylpyridines ...

La quantité de catalyseur pouvant être mise en oeuvre correspond à un rapport molaire catalyseur/hydracide formé compris entre la stoechiométrie et un excès de 10 % par rapport à la stoechiométrie.

Parmi les solvants pouvant être mis en oeuvre, on peut citer : la pyridine, les solvants aromatiques (benzène, toluène, xylène ...), les solvants aliphatiques chlorés (dichlorométhane, chloroforme, tétrachlorure de carbone, 1-2 dichloréthane ...).

Ceux-ci sont mis en oeuvre en quantité au moins suffisante pour dissoudre les réactifs.

Il est tout particulièrement intéressant d'utiliser de la pyridine, puisque celle-ci joue à la fois le rôle de catalyseur et celui de solvant.

La réaction se déroule généralement en une ou deux heures.

Le catalyseur et le solvant sont ensuite séparés de l'ester recherché ; cette opération peut être réalisée en acidifiant à froid le milieu réactionnel à l'aide d'un acide minéral tel que l'acide chlorhydrique, l'acide sulfurique, l'acide perchlorique ...

Ainsi, l'acidification peut être réalisée à une température voisine de 0° C à l'aide d'un excès d'acide par rapport à la quantité de catalyseur n'ayant pas réagi, cet excès pouvant aller jusqu'à 100 % par rapport à la stoechiométrie.

L'ester recherché est ensuite séparé par exemple par décantation, puis purifié par exemple par lavage à l'eau.

Les esters insaturés de l'acide furoïque de formule I faisant l'objet de l'invention peuvent être utilisés comme monomères pour la préparation de polymères ou de copolymères réticulables pour la fabrication de revêtements.

Les exemples suivants sont donnés à titre indicatif et ne peuvent être considérés comme une limite du domaine et de l'esprit de l'invention.

Exemple 1

Préparation du méthacrylate de 2-éthyl furoate

On introduit à une température de 25° C dans un réacteur de 2 l à double enveloppe avec circulation d'un liquide réfrigérant, muni d'un agitateur à ancre, sous débit d'azote et en atmosphère anhydre :

. 413 g (3,5 moles) de méthacrylate de monoéthylène-glycol

. et 387 g (4,9 moles) de pyridine anhydre.

On ajoute alors doucement 503 g (3,85 moles) de chlorure de furoyle de manière à ne pas dépasser une température de 15° C. Cette température est maintenue constante pendant encore 1 heure, puis on laisse reposer la masse réactionnelle pendant 10 heures à température ambiante.

Le mélange réactionnel est ensuite coulé dans une solution aqueuse contenant 4,5 moles d'acide chlorhydrique ainsi que de la glace.

La phase organique constituée par l'ester recherché est séparée par décantation ; elle est ensuite lavée avec une solution aqueuse à 10 % de bicarbonate de sodium, puis de nouveau avec de l'eau jusqu'à neutralité.

On obtient après filtration un liquide visqueux incolore.

Le rendement est de 83 % par rapport au méthacrylate de monoéthylène glycol.

Les caractéristiques du produit obtenu sont les suivantes :

. densité $d^{20} = 1,38$

. indice de réfraction $n_D^{20} = 1,494$

Les résultats de microanalyse élémentaire sont les suivants :

| | % mesuré | % théorique |
|---|---|---|
| C | 59,17 | 58,97 |
| H | 5,16 | 5,36 |
| O | 35,67 | 35,71 |

La structure de l'ester recherché est confirmée par les résultats de l'analyse spectroscopique.

L'ester est mis en solution dans du chloroforme deuterié et analysé à température ambiante à l'aide d'un appareil RMN $C^{13}$ à 22 MHz.

Les déplacements chimiques δ en ppm par rapport au tétraméthylsilane sont les suivants :

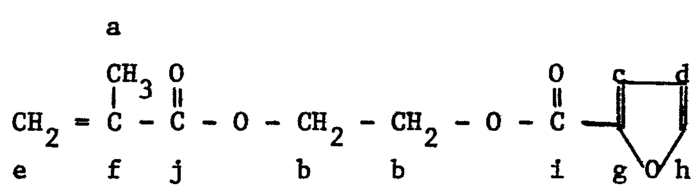

| C n° | δppm | C n° | δppm |
|---|---|---|---|
| a | 18,2 | f | 136,0 |
| b | 62,4 | g | 144,4 |
| c | 111,9 | h | 146,7 |
| d | 118,4 | i | 158,3 |
| e | 126,0 | j | 167,0 |

On analyse de même l'ester à l'aide d'un appareil RMN du

proton à 60 MHz, à température ambiante à partir d'une solution dudit ester dans du chloroforme deuterié.

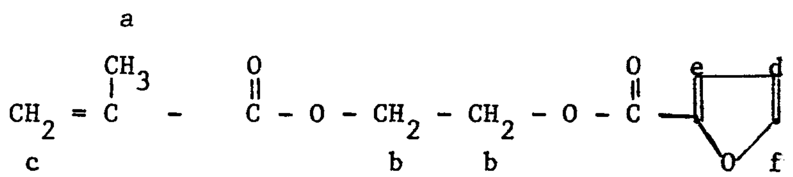

Les déplacements chimiques δ en ppm par rapport au tétraméthylsilane sont les suivants :

| H n° | | δppm | |
|---|---|---|---|
| a | | 1,92 | |
| b | | 4,5 | |
| c | doublet | 5,53 | doublet |
| c | | 6,10 | |
| d | | 6,47 | multiplet |
| e | | 7,15 | multiplet |
| f | | 7,55 | multiplet. |

L'ester analysé en spectroscopie infra-rouge présente les bandes caractéristiques suivantes :

. 1 790 $cm^{-1}$ (fonctions esters)

. 1 575 $cm^{-1}$ et 610 $cm^{-1}$ (noyau furfuryle)

. 1 635 $cm^{-1}$ (double liaison méthacrylate).

Exemple 2

Préparation d'acrylates de furoate d'isopropyle

On introduit à une température de 25° C dans un réacteur de 2 l à double enveloppe avec circulation d'un liquide réfrigérant, muni d'un agitateur à ancre, sous débit d'azote et en atmosphère anhydre :

. 455 g (3,5 moles) d'acrylate de monopropylène-glycol constitué d'environ 80 % en poids d'acrylate de 2-hydroxy-2 méthyléthyle et 20 % en poids d'acrylate de 2-hydroxy-1-méthyléthyle,

. et 387 g (4,9 moles) de pyridine anhydre.

On ajoute alors doucement 503 g (3,85 moles) de chlorure de furoyle de manière à ne pas dépasser une température de 5° C. Cette température est maintenue constante pendant encore 1 heure, puis on laisse reposer la masse réactionnelle pendant 10 heures à température ambiante.

Le mélange réactionnel est ensuite coulé dans une solution aqueuse contenant 4,5 moles d'acide chlorhydrique ainsi que de la glace.

La phase organique constituée par l'ester recherché est séparée par décantation ; elle est ensuite lavée avec une solution aqueuse à 10 % de bicarbonate de sodium, puis de nouveau avec de l'eau jusqu'à neutralité.

On obtient après filtration un liquide visqueux incolore.

Le rendement est de 80 % par rapport à l'acrylate de monopropylène glycol.

Les caractéristiques du produit obtenu sont les suivantes :

. densité $d^{25}$ = 1,16

. indice de réfraction $n_D^{20}$ = 1,492

Les résultats de microanalyse élémentaire sont les suivantes :

| | % mesuré | % théorique |
|---|---|---|
| C | 58,49 | 59,20 |
| H | 5,28 | 4,93 |
| O | 36,23 | 35,87 |

La structure des esters recherchés est confirmée par les résultats de l'analyse spectroscopique.

Le produit obtenu est mis en solution dans du chloroforme deutérié, puis analysé à température ambiante à l'aide d'un appareil RMN $C^{13}$ à 22 MHz.

Les déplacements chimiques δ en ppm par rapport au tétraméthylsilane sont les suivants :

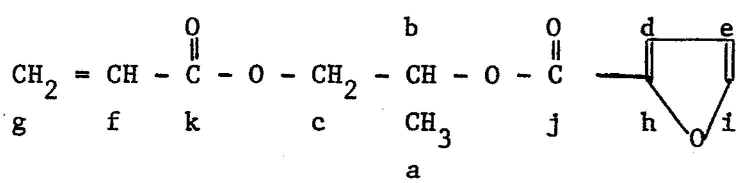

80 %

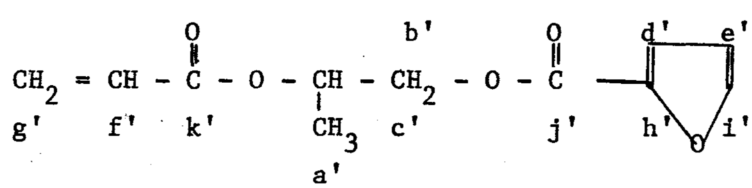

20 %

| C n° | δppm | Cn° | δppm |
|---|---|---|---|
| a | 16,5 | a' | 16,5 |
| b | 66,1 | b' | 66,2 |
| c | 69,0 | c' | 68,5 |
| d | 112,0 | d' | 112,0 |
| e | 118,2 | e' | 118,4 |
| f | 128,1 | f' | 128,6 |
| g | 131,2 | g' | 131,0 |
| h | 144,7 | h' | 144,4 |
| i | 146,7 | i' | 146,8 |
| j | 157,9 | j' | 158,1 |
| k | 165,6 | k' | 165,3 |

Le produit analysé en spectroscopie infra-rouge présente les bandes caractéristiques suivantes :

- 1725 $cm^{-1}$ (fonction esters)
- 1630 $cm^{-1}$ (doubles liaisons acrylates)
- 1575 $cm^{-1}$ et 610 $cm^{-1}$ (noyau furfuryle).

## REVENDICATIONS

1) Esters insaturés de l'acide furoïque caractérisés en ce qu'ils ont pour formule :

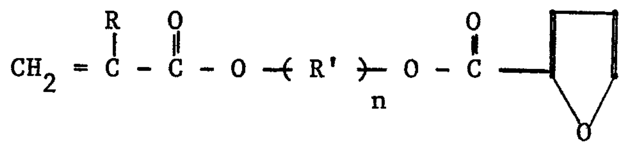

(I)

formule dans laquelle :

. R représente de l'hydrogène ou un groupe alkyle en $C_1$-$C_2$

. R' représente un radical alkylène linéaire ou ramifié en $C_1$-$C_5$

. n représente un nombre entier allant de 1 à 4 .

2) Esters selon la revendication 1 caractérisés en ce que dans la formule I R représente le groupe méthyle.

3) Esters selon la revendication 1 ou 2 caractérisés en ce que dans la formule I R' représente un radical alkylène linéaire ou ramifié en $C_2$-$C_4$.

4) Esters selon l'une quelconque des revendications précédentes caractérisés en ce que dans le formule I n est égal à 1.

5) Procédé de préparation des esters faisant l'objet de l'une quelconque des revendication précédentes caractérisé en ce qu'on fait réagir un acrylate ou alkylacrylate d'hydroxyalkyle de formule :

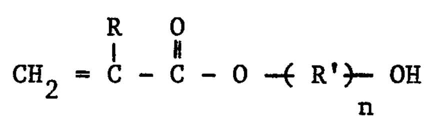

(II)

où R, R' et n ont les mêmes significations que dans la formule I avec un halogénure de furoyle selon un rapport molaire halogénure de furoyle/acrylate ou alkylacrylate de formule II compris entre la stoechiométrie et un excès de 10 % de l'un ou l'autre des réactifs par rapport à la stoechiométrie, à une température comprise entre 0 et 40° C, en présence d'un catalyseur piègeant l'hydracide formé et un solvant commun des deux réactifs.

6) Procédé selon la revendication 5 caractérisé en ce que le composé de formule II est l'acrylate ou le méthacrylate de 2 - hydroxy-1 méthyléthyle, l'acrylate ou le méthacrylate de 2-méthyléthyle, l'acrylate ou le méthacrylate de 2-hydroxyéthyle.

7) Procédé selon la revendication 6 ou 7 caractérisé en ce que l'halogénure de furoyle est le chlorure de furoyle.

8) Procédé selon l'une quelconque des revendications 5 à 7 caractérisé en ce que la température de réaction est comprise entre 5 et 20° C.

9) Procédé selon l'une quelconque des revendications 1 à 8 caractérisé en ce que le catalyseur est une amine tertiaire ne possédant pas d'hydrogène labile.

10) Procédé selon la revendication 9 caractérisé en ce que le catalyseur est de la pyridine.

11) Procédé selon l'une quelconque des revendications 1 à 10 caractérisé en ce que le rapport molaire catalyseur/hydracide formé est compris entre la stoechiométrie et un excès de 10 % par rapport à la stoechiométrie.

12) Procédé selon l'une quelconque des revendications 1 à 11 caractérisé en ce que le solvant est choisi parmi la pyridine, les solvants aromatiques, les solvants aliphatiques chlorés.

0191669

Office européen des brevets

# RAPPORT DE RECHERCHE EUROPEENNE

Numero de la demande

EP 86 40 0092

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | US-A-2 195 382 (H.R. SLAGH)<br>* En entier *<br>--- | 1-12 | C 07 D 307/68 |
| Y | GB-A-2 019 398 (INTERNATIONAL PAINT CO.)<br>* En entier *<br>----- | 1-12 | |
| | | | DOMAINES TECHNIQUES RECHERCHES (Int. Cl.4) |
| | | | C 07 D 307/00 |

Le présent rapport de recherche a été établi pour toutes les revendications

| Lieu de la recherche | Date d'achèvement de la recherche | Examinateur |
|---|---|---|
| LA HAYE | 24-04-1986 | ALLARD M.S. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03.82